(19) Europäisches Patentamt
European Patent Office
Office européen des brevets

(11) **EP 2 059 166 B1**

(12) **EUROPÄISCHE PATENTSCHRIFT**

(45) Veröffentlichungstag und Bekanntmachung des Hinweises auf die Patenterteilung:
**16.11.2016 Patentblatt 2016/46**

(21) Anmeldenummer: **07846880.8**

(22) Anmeldetag: **28.11.2007**

(51) Int Cl.:
**A61B 5/08** (2006.01)

(86) Internationale Anmeldenummer:
**PCT/EP2007/010355**

(87) Internationale Veröffentlichungsnummer:
**WO 2008/080469 (10.07.2008 Gazette 2008/28)**

(54) **VERFAHREN UND VORRICHTUNG ZUR BESTIMMUNG DER ATEMFREQUENZ**

METHOD AND DEVICE FOR THE DETERMINATION OF BREATH FREQUENCY

PROCÉDÉ ET DISPOSITIF DE DÉTERMINATION DE LA FRÉQUENCE RESPIRATOIRE

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **21.12.2006 DE 102006060819**

(43) Veröffentlichungstag der Anmeldung:
**20.05.2009 Patentblatt 2009/21**

(73) Patentinhaber: **Fresenius Medical Care Deutschland GmbH**
**61352 Bad Homburg (DE)**

(72) Erfinder:
• **ZHANG, Wei**
**97464 Niederwerrn (DE)**
• **MÜLLER, Carsten**
**97502 Euerbach (DE)**

(74) Vertreter: **Laufhütte, Dieter et al**
**Lorenz Seidler Gossel**
**Rechtsanwälte Patentanwälte**
**Partnerschaft mbB**
**Widenmayerstraße 23**
**80538 München (DE)**

(56) Entgegenhaltungen:
WO-A-03/051198   WO-A-2006/055917
DE-A1- 10 014 077   US-A1- 2006 258 921

**Beschreibung**

Die vorliegende Erfindung betrifft ein Verfahren und eine Vorrichtung zur Bestimmung der Atemfrequenz eines Patienten, welche der meßtechnischen Überwachung der Atemaktivität eines Patienten dienen.

Hierzu sind bereits mehrere unterschiedliche Methoden bekannt, um Informationen über die Atemaktivität aus unterschiedlichen physiologischen Messsignalen des Patienten zu extrahieren. So ist es möglich, die Atemaktivität eines Patienten mit folgenden Methoden abzuleiten und zu überwachen:

- über die Veränderung der Bioimpedanz aufgrund der Atembewegung des Brustkorbs, der Impedanz-Plethysmographie (IP),

- aus dem Herzratenvariabilitätssignal, da aufgrund der respiratorischen Sinusarrhythmie in der Herzrate Informationen über die Atemaktivität enthalten sind,

- aus einer optoelektrischen Messung des Blutvolumenpulses, der Photo-Plethysmographie (PPG), welche einen additiven Signalanteil aufgrund atembedingter Schwankungen des Blutdrucks enthält,

- aus Potenzialdifferenzen an der Körperoberfläche aufgrund der Herzaktivität, dem sogenannten Elektrokardiogramm (EKG),

- aus der Pulswellenlaufzeit einer Pulswelle in einer Arterie (PTT), da die Schwankung des Blutdrucks ein atembedingten Anteil aufweist und der systolische Blutdruck nahezu linear mit der Pulswellenlaufzeit korreliert ist.

Allerdings werden die Messungen der Atemfrequenz aufgrund dieser Methoden durch eine Vielzahl von Störsignalen beeinflusst. Dabei hat sich gezeigt, dass eine Bewertung der Güte der Einzelsignale aufgrund der Komplexität der Störsignale kaum möglich ist.

Dies beruht im wesentlichen auf den folgenden drei Gründen:

- Grund 1 - indirekte Messung
  Das Extrahieren der Ateminformation z.B. aus dem EKG- und PPG-Signal ist eine indirekte Messung der Atemaktivitäten und damit immer störanfällig.

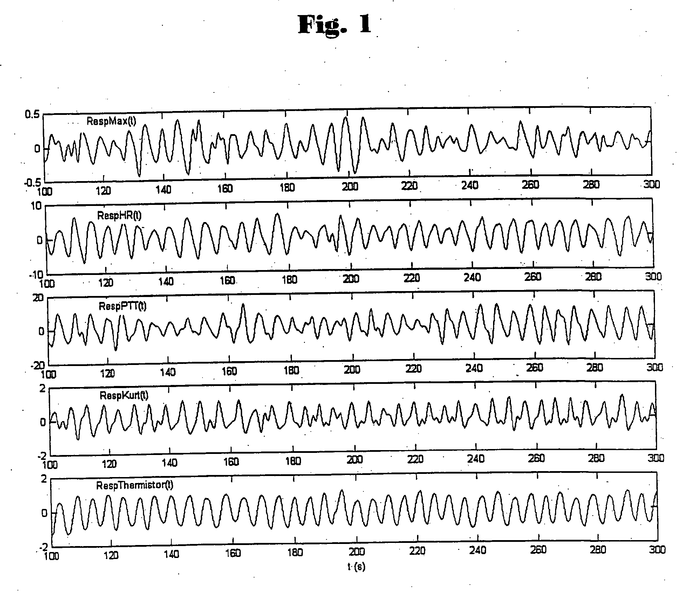

- Grund 2 - verschiedene Ausprägung der extrahierten Atemsignale
  Aus den Auswertungen von Labor- und klinischen Daten ergibt sich, dass die Ausprägung der Atemaktivitäten in den extrahierten Atemsignalen personenabhängig und auch zeitabweichend ist, siehe Fig.1. Man kann deshalb nicht einfach festlegen, dass ein extrahiertes Atemsignal definitiv besser als die anderen ist.

- Grund 3 - Artefakte
  Die extrahierten Atemsignale können unterschiedlich mit Artefakten behaftet sein, welche sich aus den unterschiedlichen Methoden ergeben können oder welche z.B. aufgrund der Bewegung des Patienten oder auch aufgrund anderer physiologischer Vorgänge entstehen können.

Zur Verbesserung der Messgenauigkeit ist es deshalb aus US 2005/0027205 bekannt, mehrere aus unterschiedlichen Messmethoden gewonnene Atemfrequenzen zu mitteln. Hierbei kommen als Verfahren Impedanz-Plethysmographie (IP) und Photo-Plethysmographie (PPG) zum Einsatz, die beide bewegungsabhängig sind. Um die durch Patientenbewegungen verursachten Artefakte zu eliminieren, wird ein spezielles mathematisches Modell zur Ermittlung von Prognosewerten eingesetzt, das für beide Messkanäle jeweils separat eine Vorhersage macht. Die Vorhersage basiert jeweils nur auf vergangen Messwerten für den jeweiligen Kanal sowie einem Faktor, der pauschal übliche Abweichungen in der Frequenz berücksichtigt, d.h. das Ausgangssignal jedes Kanals wird geglättet und extrapoliert. Die gemessenen Frequenzen werden nun mit den prognostizierten Werten verglichen und über diese Differenz die Gewichte für die Mittelung der Frequenzen bestimmt. Die Gewichtung beruht dabei aber allein auf der Differenz zum Modell für den jeweiligen Messkanal. Dieses Vorgehen setzt deshalb voraus, dass das Modell die Realität besser beschreibt als die Messungen, da keine Rückkoppelung aus den Messergebnissen auf die Modellstruktur erfolgt. Hierdurch können nur zeitweise Störungen, wie sie etwa durch Patientenbewegungen entstehen, gedämpft werden, während permanente bzw. systematische Störeinflüsse nicht eliminiert werden können. Insbesondere Fehler aufgrund von physiologischen Störfaktoren wie z.B. Mayerwellen werden dadurch aber verschleppt übernommen und verfälschen die durch ein solches System ermittelte Atemfrequenz weiterhin erheblich. Zudem ist die Berechnung über die Prognosemodelle aufwendig und kompliziert.

Aufgabe der vorliegenden Erfindung ist es deshalb, ein verbessertes Verfahren zur Bestimmung der Atemfrequenz eines Patienten zur Verfügung zu stellen, welches die Zuverlässigkeit der bestimmten Atemfrequenz auf einfache Art und Weise erhöht und insbesondere auch physiologische Störfaktoren eliminieren kann. Erfindungsgemäß wird diese Aufgabe von einem Verfahren zur Bestimmung der Atemfrequenz eines Patienten gemäß Anspruch 1 gelöst. Ein solches Verfahren enthält die Schritte des Bestimmens von mindestens zwei zeitabhängigen Atemsignalen $s_i(t)$ ($i$ = 1, 2, ...) durch mindestens zwei unterschiedliche Methoden und des Bestimmens der sich aus den mindestens zwei zeitabhängigen Atemsignalen $s_i(t)$ ($i$ = 1, 2, ...) ergebenden jeweiligen momentanen Atemfrequenzen $f_i(n)$ ($i$ =1, 2, ...) und des Bestimmens einer mittleren Atemfrequenz $f(n)$ durch eine gewichtete Mittelung der Atemfrequenzen $f_i(n)$ ($i$ = 1, 2,...). Bei dieser Mittelung hängen die Gewichte $k_i(n)$ ($i$ = 1, 2, ...) der einzelnen Atemfrequenzen $f_i(n)$ ($i$ =1, 2, ...) von einer Differenz zwischen den jeweiligen Atemfrequenzen $f_i(n)$ ($i$ = 1, 2,...) und einem Schätzwert $f_s(n)$ ab, der auf Grundlage von mindestens zwei Atemsignalen $s_i(t)$ ($i$ = 1, 2, ...) bestimmt wird. Die Gewichtung erfolgt also nicht mehr für jeden Kanal getrennt, sondern beruht auf der Differenz der jeweiligen Atemfrequenzen $f_i(n)$ ($i$ = 1, 2, ...) mit einem Prognosewert, der auf der Grundlage von Daten aus mehreren Kanälen bestimmt wird. Da sich Störungen üblicherweise unterschiedlich auf die unterschiedlichen zeitabhängigen Atemsignale $s_i(t)$ ($i$ = 1, 2, ...) und damit auch auf die daraus ermittelten Atemfrequenzen $f_i(n)$ ($i$ = 1, 2, ...) auswirken, können über eine solche Gewichtung Störsignale und Fehler in den einzelnen Atemsignalen unterdrückt werden. Liegt eine Störung in nur einem Atemsignal und damit nur einer Atemfrequenz vor, wird die Differenz zwischen dieser Atemfrequenz und dem Schätzwert $f_s$ groß sein, was bei der Mittelung wiederum zu einem geringen Gewicht dieser Atemfrequenz führt. Hierdurch ergibt sich eine Rückkopplung auf die Gewichtung der einzelnen Kanäle, durch die auch systematische bzw. permanente Störeinflüsse eliminiert werden können. Insbesondere ist es so möglich, den Einfluss von physiologischen Störfaktoren wie z.B. Mayerwellen zu eliminieren.

Vorteilhafterweise wird dabei der Schätzwert $f_s(n)$ auf Grundlage einer vorangegangenen bereits bestimmten mittleren Atemfrequenz $f(n\text{-}1)$ bestimmt. Insbesondere können so die Gewichte vorteilhafterweise aus der Differenz zwischen den aktuellen über die jeweiligen Kanäle gemessenen Atemfrequenzen $f_i(n)$ ($i$ = 1, 2,...) und dem zuletzt bestimmten Mittelwert $f(n\text{-}1)$ bestimmt werden. Ist diese Differenz groß, wird dem betreffenden Kanal ein kleines Gewicht zugeordnet und umgekehrt. Abweichungen der einzelnen Werte werden so immer auf das Gesamtsystem bezogen, so dass über diese Rückkopplung der Systemstruktur des gesamten Systems auch systematische Fehler eliminiert werden können. Weiterhin vorteilhafterweise wird der Schätzwert $f_s(n)$ insbesondere zur Initialisierung durch eine Kombination von Frequenzinformationen aus mindestens zwei zeitabhängigen Atemsignalen $s_i(t)$ ($i$ = 1, 2,...) oder durch Bilden eines Mittelwertes der aktuellen Atemfrequenzen $f_i(n)$ ($i$ = 1, 2,...) bestimmt. Da gerade zu Anfang keine zuverlässigen Schätzwerte aus vergangenen Messungen vorliegen, kann entweder auf (im Normalfall ungewichtete) Mittelwerte der aktuellen Messwerte zurückgegriffen werden oder aber durch eine Kombination von Frequenzinformationen ein Schätzwert zur Verfügung gestellt werden. Die Verwendung von Frequenzinformationen ist dabei relativ rechenaufwendig, liefert dafür aber auch genauere Ergebnisse. Dies ist insbesondere zur Initialisierung von Vorteil, kann aber auch dann zum Einsatz kommen, wenn aufgrund von starken Störungen während des Betriebs anders keine Werte ermittelt werden können.

Vorteilhafterweise werden die mindestens zwei zeitabhängigen Atemsignale $s_i(t)$ ($i$ = 1, 2, ...) aus gemessenen physiologischen Signalen ermittelt. Dabei können die zeitabhängigen Atemsignale $s_i(t)$ ($i$ = 1, 2, ...) durch unterschiedliche Methoden aus einem oder mehreren gemessenen physiologischen Signalen ermittelt werden, was die Zuverlässigkeit des Endergebnisses erhöht.

Dabei bilden die gemessenen physiologischen Signale vorteilhafterweise eine Auswahl aus folgenden Signalen:

- Bioimpedanzsignal,
- Herzratenvariabilitätssignal,
- photoplethysmographisches Signal (PPG-Signal),
- Quellenstatistiksignal des EKGs,
- Pulswellenlaufzeitsignal (PTT-Signal).

So ergibt sich eine Vielzahl von unterschiedlichen physiologischen Signalen, welche gemessen und zur Ermittlung der zeitabhängigen Atemsignale $s_i(t)$ ($i$ = 1, 2,...) genutzt werden können.

Vorteilhafterweise geschieht dabei die Ermittlung der mindestens zwei zeitabhängigen Atemsignale $s_i(t)$ ($i$ = 1, 2, ...) aus den gemessenen physiologischen Signalen durch einen Bandpassfilter. Da die physiologischen Signale üblicherweise nicht nur Information zur Atemaktivität, sondern auch andere Information z. B. zur Herzrate enthalten, können durch einen Bandpassfilter diese nicht gewünschten Informationen ausgefiltert werden, so daß sich aus den physiologischen Signalen die zeitabhängigen Atemsignale $s_i(t)$ ($i$ = 1, 2,...) ergeben.

Dabei lässt der Bandpassfilter vorteilhafterweise Frequenzen in einem Bereich von ca. 0,12 Hz bis 0,42 Hz passieren, während andere Frequenzen, welche außerhalb dieses Bereichs der Atemfrequenzen liegen, vom Bandpassfilter unterdrückt werden.

Vorteilhafterweise umfaßt das erfindungsgemäße Verfahren weiterhin den Schritt der Bestimmung einer momentanen Atemfrequenz $f_i(k)$ aus einem zeitabhängigen Atemsignal $s_i(t)$ ($i$ = 1, 2, ...) durch Bestimmung des Zeitindexes

$t_{max}$ (k) der Maxima des zeitabhängigen Atemsignals $s_i(t)$ (i = 1, 2, ...). Aus dem zeitabhängigen Atemsignal $s_i(t)$ (i = 1, 2,...) kann so durch die Bestimmung seiner Maximal bzw. durch die Bestimmung der Zeitindizes der Maxima die Atemfrequenz auf einfache Art und Weise ermittelt werden. Vorteilhafterweise geschieht dabei die Bestimmung der momentanen Atemfrequenz durch die Bestimmung des zeitlichen Abstandes $t_{max}(k) - t_{max}(k-1)$ zwischen benachbarten Maxima des zeitabhängigen Atemsignals. Der zeitliche Abstand zwischen zwei aufeinander folgenden Maxima des zeitabhängigen Atemsignals ist dabei umgekehrt proportional zur momentanen Atemfrequenz $f_i(k)$. Vorteilhafterweise wird aus drei Atemsignalen die momentane Atemfrequenz bestimmt: $f_{hr}(m)$, $f_{amp}(n)$, $f_{ptt}(k)$. Vorteilhafterweise erfolgt dabei eine Konsistenzprüfung der Zeitindizes m, n und k. Die Zeitindizes müssen sich dafür innerhalb eines vorgegebenen Zeitfensters befinden. Für das Zeitfenster kann man beispielsweise 50% der aktuellen Atemperiode verwenden.

Die vorliegende Erfindung umfasst weiterhin ein Verfahren, bei welchem eine Konsistenzprüfung der Atemfrequenzen $f_i(n)$ (i = 1, 2,...) durchgeführt wird. So können fehlerhafte Signale identifiziert und bei der Bestimmung der Atemfrequenz f unterdrückt werden. Vorteilhafterweise geschieht dies in dem oben beschriebenen Verfahren vor der gewichteten Mittelung der Atemfrequenzen $f_i(n)$ (i = 1, 2, ...). Für den Fachmann ist es aber offensichtlich, dass eine solche Konsistenzprüfung auch unabhängig von der konkreten Mittelung von großem Vorteil ist.

Vorteilhafterweise erfolgt die Konsistenzprüfung durch einen Vergleich der Atemfrequenzen $f_i(n)$ (i =1, 2, ...) untereinander. Dies ermöglicht auf einfache Art und Weise eine Prüfung der Konsistenz der einzelnen Atemfrequenzen $f_i(n)$ (i =1, 2, ...), so dass inkonsistente Werte aussortiert werden können und die Qualität des Signals aus diesen Differenzen bestimmt werden kann. Je mehr Übereinstimmungen zwischen den unterschiedlichen Atemfrequenzen $f_i(n)$ (i = 1, 2, ...) bei der Konsistenzprüfung aufgefunden werden, desto höher wird die Signalgüte bewertet.

Vorteilhafterweise wird weiterhin die Differenz zwischen den jeweiligen Atemfrequenzen $f_i(n)$ (i = 1, 2, ...) mit einer erlaubten Toleranz $\Delta$ verglichen. So werden geringfügige Abweichungen bei der Konsistenzprüfung ignoriert, während große Abweichungen eine Inkonsistenz zwischen den einzelnen Werten der Atemfrequenzen $f_i(n)$ (i = 1, 2, ...) anzeigen.

Vorteilhafterweise werden dabei zu der gewichteten Mittelung der Atemfrequenzen $f_i(n)$ (i = 1, 2, ...) nur solche Atemfrequenzen herangezogen, welche die Konsistenzprüfung bestehen. So können Fehler gleich von vornherein unterdrückt werden und beeinflussen das Endergebnis nicht mehr. Zudem lässt sich aus der Zahl der die Konsistenzprüfung bestehenden Atemfrequenzen die Signalgüte bewerten.

Weiterhin vorteilhafterweise umfasst die vorliegende Erfindung ein Verfahren, bei dem die Signalgüte insbesondere wie oben beschrieben über eine Konsistenzprüfung bestimmt und gegebenenfalls angezeigt wird. Für den Fachmann ist dabei offensichtlich, dass eine solche Bestimmung der Signalgüte wichtige Informationen zur Beurteilung der Messergebnisse liefert und auch unabhängig von den oben beschriebenen Merkmalen des Verfahrens von großem Vorteil ist.

Die vorliegende Erfindung umfasst weiterhin ein Verfahren mit folgenden Schritten: Die Erzeugung von mindestens zwei Frequenzsignalen $FT_i(f)$ (i = 1, 2,...) durch Transformation von mindestens zwei zeitabhängigen Atemsignalen $s_i(t)$ (i = 1, 2, ...) in den Frequenzraum, sowie das Bestimmen eines Frequenzsignals $FT(f)$ durch eine Kombination der Frequenzsignale $FT_i(f)$ (i = 1, 2, ...), wobei eine Atemfrequenz f auf Grundlage des Frequenzsignals $FT(f)$ bestimmt wird. Die Transformation der zeitabhängigen Atemsignale $s_i(t)$ (i = 1, 2, ...) in den Frequenzraum kann dabei durch eine Fourier-Transformation geschehen, und vorteilhafterweise über eine Fast-Fourier-Transformation (FFT). So ergeben sich die Frequenzspektren der unterschiedlichen Atemsignale, welche dann zur Bestimmung des Frequenzsignals $FT(f)$ verwendet werden können. Auch dies ermöglicht eine einfache und sichere Unterdrückung von Störsignalen und Fehlern im Endergebnis. Für den Fachmann ist dabei offensichtlich, dass es sich bei dieser Methode um eine von der oben beschriebenen Mittelung im Zeit-Raum unabhängige Methode handelt, welche aber vorteilhafterweise mit dieser z.B. zur Initialisierung der gewichteten Mittelung oder zur Überbrückung bei starken Störungen kombiniert werden kann.

Vorteilhafterweise wird bei der Kombination der Frequenzsignale dabei das Frequenzsignal $FT(f)$ durch eine Mittelung der Frequenzsignale $FT_i(f)$ (i = 1, 2,...) bestimmt. Vorteilhafterweise wird dabei der geometrische Mittelwert berechnet.

Die Atemfrequenz f wird nun vorteilhafterweise durch Peak-Detektion des Frequenzsignals $FT(f)$ bestimmt, so dass sich direkt aus dem Frequenzsignal die mittlere Atemfrequenz f ableiten lässt.

Alternativ kann die Atemfrequenz f jedoch auch durch Rücktransformation des Frequenzsignals $FT(f)$ und eine Auswertung des sich ergebenden Signals $s(t)$ bestimmt werden. Diese Auswertung kann dann wie oben bereits beschrieben durch eine Bestimmung der Maxima des Signals $s(t)$ erfolgen.

Damit stehen zwei einfache Methoden zur Verfügung, um die Atemfrequenz f aus dem Frequenzsignal $FT(f)$ zu bestimmen.

Weiterhin vorteilhafterweise werden bei dem erfindungsgemäßen Verfahren die mindestens zwei zeitabhängigen Atemsignale $s_i(t)$ (i = 1, 2, ...) aus einem PPG-Signal und einem EKG-Signal gewonnenen. Diese beiden Signale enthalten eine Vielzahl von Informationen zur Atemfrequenz und bilden so eine zuverlässige Grundlage zur Bestimmung der mindestens zwei zeitabhängigen Atemsignale $s_i(t)$ (i = 1, 2,...) durch unterschiedliche Methoden.

Vorteilhafterweise bilden die mindestens zwei zeitabhängigen Atemsignale $s_i(t)$ (i = 1, 2, ...) des erfindungsgemäßen Verfahrens eine Auswahl aus folgenden Signalen:

- ein aus der Herzfrequenz bestimmtes Atemsignal $s_{HR}(t)$,
- ein aus dem PPG-Signal bestimmtes Atemsignal $s_{PPG}(t)$,
- ein aus dem PTT-Signal bestimmtes Atemsignal $s_{PTT}(t)$,
- ein aus der Kurtosis des EKG-Signals bestimmtes Atemsignal $s_{kurt}(t)$.

All diese Atemsignale können dann ausgewertet und zur Bestimmung der Atemfrequenz *f* des Patienten genutzt werden.

Vorteilhafterweise werden dabei alle vier Atemsignale in dem erfindungsgemäßen Verfahren verwendet, um eine möglichst hohe Zuverlässigkeit und Genauigkeit des Ergebnisses zu erreichen. Insbesondere bei der Verwendung der Konsistenzprüfung und der Bestimmung der Signalgüte ist dabei eine hohe Anzahl an Atemsignalen von Vorteil.

Die vorliegende Erfindung umfaßt weiterhin eine Vorrichtung zur Bestimmung der Atemfrequenz eines Patienten mittels eines der oben beschriebenen Verfahren. Hierdurch ergeben sich offensichtlich die gleichen Vorteile, wie sie bereits anhand des Verfahrens dargestellt wurden. Eine solche Vorrichtung umfaßt dabei insbesondere Sensoren zur Messung von physiologischen Signalen, aus welchen die mindestens zwei zeitabhängigen Atemsignale bestimmt werden können, sowie ein Mittel zur Datenverarbeitung, welche so ausgeführt sind, daß sie das erfindungsgemäße Verfahren durchführen.

Weiterhin vorteilhafterweise umfaßt die vorliegende Erfindung eine Vorrichtung zur Bestimmung der Atemfrequenz eines Patienten insbesondere zur Durchführung des erfindungsgemäßen Verfahrens, mit einer separaten Sensoreinheit zur Messung der physiologischen Signale, aus welchen die mindestens zwei zeitabhängigen Atemsignale bestimmt werden können, und einer Recheneinheit zur Auswertung der von der Sensoreinheit übertragenen Daten. Dadurch, das zumindest ein Großteil des Verfahrens zur Bestimmung der Atemfrequenz eines Patienten nicht in der Sensoreinheit, sondern in der Recheneinheit durchgeführt wird, muß die zur Durchführung der in der Sensoreinheit durchgeführten Verfahrensschritte benötigte Rechenleistung der Sensoreinheit nicht allzu groß dimensioniert werden, was einen kostengünstigen und platzsparenden Aufbau ermöglicht. Durch die separate Sensoreinheit ist eine besonders einfache Bedienung der erfindungsgemäßen Vorrichtung möglich, wobei sich besondere Vorteile insbesondere bei Verwendung des erfindungsgemäßen Verfahrens ergeben. Für den Fachmann ist jedoch jedenfalls offensichtlich, daß sich ebenfalls Vorteile bei Verwendung eines Verfahrens nach dem Stand der Technik ergeben.

Weiterhin vorteilhafterweise werden die von der Sensoreinheit erzeugten Daten drahtlos an die Recheneinheit übertragen. Hierdurch ist keine komplizierte Verkabelung notwendig, was wiederum die Benutzerfreundlichkeit sowie die Bediensicherheit der erfindungsgemäßen Vorrichtung erhöht.

Weiterhin vorteilhafterweise wird die Sensoreinheit am Handgelenk des Patienten befestigt. Eine solche z. B. als Armbandgerät ausgeführte Sensoreinheit ermöglicht eine besonders einfache Bedienung, welche auch für den Patienten wenig belastend ist. Zur Datenübertragung kann dabei jede bekannte Art der drahtlosen Übertragung verwendet werden, wobei insbesondere eine Funkübertragung der Daten von Vorteil ist. Die Daten werden dabei drahtlos von der Sensoreinheit an die Recheneinheit übertragen, welche z. B. in einem Gerät zur Behandlung oder zum Monitoring des Patienten angeordnet ist.

In der Sensoreinheit können bereits Teile des Verfahrens zur Bestimmung der Atemfrequenz durchgeführt werden, so daß weiterverarbeitete Daten an die Recheneinheit übermittelt werden. So muß zwar eine gewisse Rechenleistung in der Sensoreinheit zur Verfügung gestellt werden, dafür sind jedoch die zu übertragenden Datenmengen von Sensoreinheit zur Recheneinheit kleiner, so daß die Datenübertragungsmittel von der Sensoreinheit zur Recheneinheit weniger aufwendig dimensioniert werden können. Insbesondere bei der Verwendung einer drahtlosen Übertragung hat dies erhebliche Vorteile.

Vorteilhafterweise werden dabei die mindestens zwei zeitabhängigen Atemsignale in der Sensoreinheit aus den physiologischen Signalen bestimmt und daraufhin an die Recheneinheit übermittelt. Die Auswertung mittels Bandpaß und die nachfolgenden Schritte des erfindungsgemäßen Verfahrens erfolgen dann durch die Elektronik der Recheneinheit.

Selbstverständlich ist es auch möglich, weitere Schritte des erfindungsgemäßen Verfahrens in der Sensoreinheit durchzuführen, wobei hier jedoch zu Beachten ist, daß für die weitere Auswertung eine bestimmte Rechenleistung (Prozessorleistung) erforderlich ist, so daß aufwendige Hardware vorzugsweise nicht in der Sensoreinheit, sondern in der Recheneinheit angeordnet ist. Die Schnittstelle kann jedoch prinzipiell beliebig gewählt werden.

Weiterhin vorteilhafterweise weist die erfindungsgemäße Vorrichtung Sensoren zur Messung des EKG-Signals und des PPG-Signals auf. Aus diesen beiden physiologischen Signalen lassen sich die mindestens zwei zeitabhängigen Atemsignale des erfindungsgemäßen Verfahrens bestimmen, wobei etwaige Fehler in den einzelnen Signalen durch die erfindungsgemäße Mittelung eliminiert werden können. Weiterhin vorteilhafterweise wird dabei aus dem EKG-Signal und dem PPG-Signal die Herzrate, die Pulsamplitude und die Pulswellenlaufzeit bestimmt. Hierdurch stehen drei unterschiedliche zeitabhängige Atemsignale zur Verfügung, durch deren erfindungsgemäße Mittelung auch systematische Fehler in den Ausgangssignalen eliminiert werden können.

Vorteilhafterweise ist die Recheneinheit dabei Teil eines medizinischen Geräts, insbesondere eines medizinischen Gerätes zur extrakorporalen Blutbehandlung wie z. B. einer Dialysemaschine, einer Hämoflitrationsmaschine oder einer Hämodiafiltrationsmaschine. Selbstverständlich kann die Datenübertragung und weitere erfindungsgemäße Auswertung der Daten aber auch in Verbindung mit einem beliebigen anderen medizinischen Gerät erfolgen.

Alternativ kann die Recheneinheit der erfindungsgemäßen Vorrichtung auch Teil eines Rechnernetzwerkes z. B. eines Krankenhauses oder einer Dialyseklinik sein. Dies hat den Vorteil, daß die aufwendige Hardware zur Auswertung der von der Sensoreinheit übertragenen Daten im Rechnernetzwerk des Krankenhauses oder der Dialyseklinik untergebracht werden kann.

Die vorliegende Erfindung wird nun anhand von Zeichnungen näher beschrieben.

Dabei zeigen:

Figur 1:    vier extrahierte Atemsignale und ein mit einem Thermistor gemessenes Atemsignal,

Figur 2:    Frequenzspektren der vier extrahierten Atemsignale, den geometrischen Mittelwert der vier Frequenzspektren und das Frequenzspektrum des Thermistorsignals,

Figur 3:    die Struktur eines Ausführungsbeispiels der erfindungsgemäßen Methodenkombination,

Figur 4:    ein mit einem Thermistor gemessenes Atemsignal als Referenz sowie drei extrahierte Atemsignale und

Figur 5:    die aus einzelnen Kanälen ermittelten Atemfrequenzen sowie die erfindungsgemäß aus der Kombination ermittelte Atemfrequenz im Vergleich zur Atemfrequenz aus dem Thermistor-Signal.

Aus dem Stand der Technik sind neben der direkten Atemüberwachung über einen Thermistor, welcher von den Patienten aber als sehr störend empfunden wird, noch folgende Methoden für die indirekte Atemüberwachung bekannt:

-    Atemüberwachung über die Bioimpedanzmessung [1]
    Beim Einatmen erweitert sich die Brust und die Impedanz steigt. Beim Ausatmen zieht sich die Brust zusammen und die Impedanz sinkt. Wird ein konstanter Wechselstrom durch den Thorax geleitet, so kann über zwei EKG-Elektroden eine atemabhängige Spannung gemessen werden.

    -    Atemaktivität aus dem Herzratenvariabilitätssignal [2]
    -    Atemaktivität aus dem photoplethysmographischen Signal (PPG-Signal) [3]
    -    Atemaktivität aus der Quellenstatistik des EKG [4]
    -    Atemaktivität aus der Pulswellenlaufzeit [5]

In dem Ausführungsbeispiel der vorliegenden Erfindung wird nun eine Verbesserung der Zuverlässigkeit der aus dem EKG- und PPG-Signal extrahierten Ateminformation durch die Kombination der bekannten Methoden entweder im Zeit- und Frequenzbereich erreicht.

2. Physiologische Grundlagen

Im folgenden wird aus dem physiologischen Aspekt erläutert, warum das EKG- und PPG-Signal Informationen über die Atmung enthält.

2.1 Respiratorische Sinusarrhytmie (RSA)

-    Die Abhängigkeit der Herzfrequenz von der Respiration wird als Respiratorische Sinusarrhytmie (RSA) bezeichnet.

    -    Während der Inspiration eine Zunahme der Herzfrequenz
    -    Während der Exspiration eine Abnahme der Herzfrequenz

-    Die RSA wird vor allem durch die wechselnde Aktivität des Nervus vagus vermittelt. So kann die respiratorische Sinusarrhythmie durch Atropingabe oder Vagotomie unterbrochen werden.

- Einflüsse auf die atmungsabhängige Herzfrequenzvariabilität: z. B. pulmonale, vasukäre und kardiale Dehnungsrezeptoren und respiratorische Zentren im Hirnstamm, unterschiedliche Baroreflex-Sensivität in den jeweiligen Phasen des Atemzyklus.

- Aufgrund einer inspiratorischen vagalen Inhibition ergeben sich Fluktuationen der Herzfrequenz mit derselben Frequenz wie die Atmung.

- Die inspiratorische Inhibitition wird primär durch den Einfluss des medullären respiratorischen auf das medulläre kardiovaskuläre Zentrum verursacht.

- Zusätzlich sind periphere Reflexe aufgrund hämodynamischer Veränderungen und thorakaler Dehnungsrezeptoren verantwortlich.

- Entsprechend sind auch Fluktuationen des Blutdrucks (Traube-Hering Wellen) mit derselben Frequenz bekannt.

Andere periodische Fluktuationen der Herzfrequenz, neben der respiratorischen Sinusarrhythmie, sind die Barorezeptorreflex- und die thermoregulatorisch bedingten Herzfrequenzveränderungen:

- Der sogenannte 10-Sekunden-Rhythmus der Herzfrequenz ist durch Eigenoszillationen des vasomotorischen Teils der Baroreflex-Schleife bedingt.

- Diese intrinsischen Oszillationen resultieren aus dem negativen Baroreflex-Feedbacksystem und werden begleitet von synchronen Fluktuationen des Blutdrucks (Mayer-Wellen).

- Die Frequenz dieser Fluktuationen wird festgelegt durch die Zeitverzögerung des Systems, die bei erhöhtem Sympathikotonus zu- und bei Sympathikus- oder Parasymphatikusblockaden abnimmt.

- Der periphere Gefäßwiderstand zeigt intrinsische Oszillationen mit einer niedrigen Frequenz.

- Diese Fluktuationen können durch eine thermische Hautstimulation hervorgerufen werden und werden somit als Reaktion auf thermoregulatorisch notwendige Veränderungen des dermalen Blutflusses angesehen.

- Diese periodischen Veränderungen des peripheren Widerstands werden begleitet von Oszillationen des Blutdrucks und der Herzfrequenz.

2.2 Atembedingte Schwankungen im Blutdruck

Der Blutdruck schwankt, abhängig von der Atmung, um einen mittleren Wert. Als Ursache werden mechanische Auswirkungen der Atmung auf den Blutdruck vermutet. Mayer fand weitere Blutdruckoszillationen, deren Frequenzen niedriger waren als die der Atmung. Sie entstehen durch Änderungen des peripheren Gefäßtonus mit einer Periodik von ca. 10-20 sec. (0,1 Hz) und werden "Mayerwellen" genannt. Die physiologischen Blutdruckveränderungen werden in Schwankungen I., II. und III. Ordnung unterteilt:

I. Ordnung: Änderung durch Systole und Diastole,
II. Ordnung: Änderungen in Abhängigkeit von der Atmung und
III. Ordnung: Mayerwellen (0,1 Hz).

Zusätzlich sind Blutdruckschwankungen niedrigerer Frequenz (<0,04 Hz) bekannt.
In der folgenden Tabelle werden die Schwankungen im Blutdruck mit den entsprechenden Ursachen zusammengefasst:

Tabelle 1: Rhythmus im Blutdruck und mögliche Ursache

| Blutdruckschwankung | Frequenzbereich (Hz) | mögliche Ursachen |
|---|---|---|
| I. Ordnung | 0,5~2,0 | Herzkontraktion |
| II. Ordnung | 0,15~0,40 | Atmung - mechanische Auswirkungen der Atmung auf den Blutdruck |

(fortgesetzt)

| Blutdruckschwankung | Frequenzbereich (Hz) | mögliche Ursachen |
|---|---|---|
| III. Ordnung | 0,04~0,15 | Mayerwellen - Der Sympathikus vermittelt einen Teil dieser Fluktuationen. Die LF-Power unterliegt der Regulation durch Baroreflex und homorale Einflüsse. |
| | < 0,04 | Die Oszillationen reflektieren die Interaktion verschiedener Kontrollmechanismen, z. B. der Thermoregulation und des Renin-Angiotensin-Systems, der endothelialen Funktion. |

3. Extrahierung der Atemaktivität aus dem PPG und EKG

3.1 Atemaktivität aus dem Herzfrequenzsignal

Aufgrund der respiratorischen Sinusarrhythmie sind das EKG- und PPG-Signal durch die Atmung frequenzmoduliert. Diesbezüglich ist das PPG-Signal durch

$$PPG(t) = PPG\big(\omega_{Herz} \cdot s(\omega_{Resp} \cdot t) \cdot t\big)$$

gegeben, wobei
$\omega_{Herz}$ die Herzfrequenz und
$s(\omega_{Resp} \cdot t)$ das Atemsignal mit der Atemfrequenz $\omega_{Resp}$ darstellen.

Die Frequenzmodulation durch die Atmung kann demoduliert werden, indem zuerst die momentane Herzfrequenz aus dem EKG-Signal oder aus dem PPG-Signal auf "beat-to-beat" Basis ermittelt wird. Danach wird das Herzfrequenzvariabilitätssignal und damit das zeitliche Atemsignal $s_{HR}(t)$ mit Hilfe eines Bandpassfilters von 0,12 Hz - 0,42 Hz extrahiert.

3.2 Atemaktivität aus dem PPG-Signal

Infolge von atembedingten Schwankungen im Blutdruck geht die Atemaktivität in Form eines additiven Signalanteils in das PPG-Signal ein. Der Atemrhythmus ist im PPG-Signal ausgeprägt und dargestellt durch

$$PPG(t) = PPG\big(\omega_{Herz} \cdot s(\omega_{Resp} \cdot t) \cdot t\big) + k_{ppg} \cdot s(\omega_{Resp} \cdot t),$$

wobei $k_{ppg}$ die Stärke der additiven Ausprägung von $s(\omega_{Resp} \cdot t)$ im PPG-Signal darstellt.
Zur Gewinnung der additiven Atemsignale kann zuerst die Hüllkurve des PPG-Signals durch die "beat-to-beat" Ermittlung der lokalen Maxima oder Minima im PPG-Signal gebildet werden und danach das zeitliche Atemsignal $s_{PPG}(t)$ mit Hilfe des Bandpassfilters extrahiert werden.

3.3 Atemaktivität aus dem PTT-Signal

Da einerseits die Schwankung im Blutdruck einen atembedingten Anteil aufweist und andererseits der systolische Blutdruck nahezu linear mit der PTT korreliert, ist auch in der PTT Ateminformation enthalten. Dies bewirkt, dass die PTT einen additiven Atemanteil aufweist. Das PTT-Signal kann deshalb durch

$$PTT(t) = PTT_{sBP}(t) + k_{ptt} \cdot s(\omega_{Resp} \cdot t)$$

angegeben werden, wobei
$PTT_{sBP}(t)$ den systolischen blutdruckbedingten Anteil in der PTT und
$k_{ptt}$ die Stärke der additiven Ausprägung von $s(\omega_{Resp} \cdot t)$ im PTT-Signal bezeichnen.

Mit Hilfe des Bandpassfilters kann Atemaktivität aus dem PTT-Signal extrahiert werden.

3.4. Atemaktivität aus der Kurtosis des EKGs

Die Grundlage dieser Methode bildet die Annahme, dass der Übertragungsweg der elektrischen Signale vom Herzen über den Brustkorb bis an die Hautoberfläche als ein lineares zeitvariantes System betrachtet werden kann, dessen Eigenschaften durch den Zustand des Körpers vorgegeben werden. Eine Eigenschaft des Systems ist dabei die Impedanz des Brustkorbs, welche durch die Atmung verändert wird. Diese zeitlichen Variationen des Systems sollen durch die Kurtosis sichtbar gemacht werden. Der Kurtosiswert wird nach folgender Formel berechnet:

$$Kurtosis = \frac{1}{T} \sum_{t=1}^{T} \left[ \frac{x_1 - \bar{x}}{\sqrt{\frac{1}{T} \sum_{t=1}^{T} (x_1 - \bar{x})^2}} \right]^4$$

Die Vorgehensweise zur Extrahierung des Atemrhythmus aus dem EKG mit der Kurtosis-Methode lässt sich in folgende Schritte aufteilen:

1. Entfernen des Baseline-Drifts im EKG-Signal,
2. Auffinden der R-Zacken: Der EKG-Signalverlauf zwischen zwei aufeinanderfolgenden R-Zacken bildet ein Intervall,
3. Kurtosis-Berechnung: Für jedes definierte Intervall wird die Kurtosis nach der oben angegebenen Formel berechnet und mit dem zugehörigen Zeitpunkt gespeichert,
4. Bilden einer Hüllkurve über die berechneten Kurtosiswerte,
5. Das zeitliche Atemsignal $s_{kurt}(t)$ entsteht durch die Filterung der Hüllkurve mit Hilfe des Bandpassfilters.

4. Methodenkombination

Wie bereits eingangs erwähnt, sind im Blutdruck und in der Herzfrequenz nicht nur der Atemrhythmus ausgeprägt, sondern auch andere Störrhythmen wie z. B. Mayerwellen und Fluktuationen durch den Gefäßtonus und die Thermoregulation, die sich im Frequenzbereich von 0,0 Hz ~ 0,15 Hz befinden. Da solche Störrhythmen sich teilweise mit dem Atemrhythmus im Frequenzbereich überlagern, können sie auch in den aus dem PPG und EKG extrahierten Atemsignalen vorhanden sein. Dadurch kann die Atemmessung verfälscht werden.

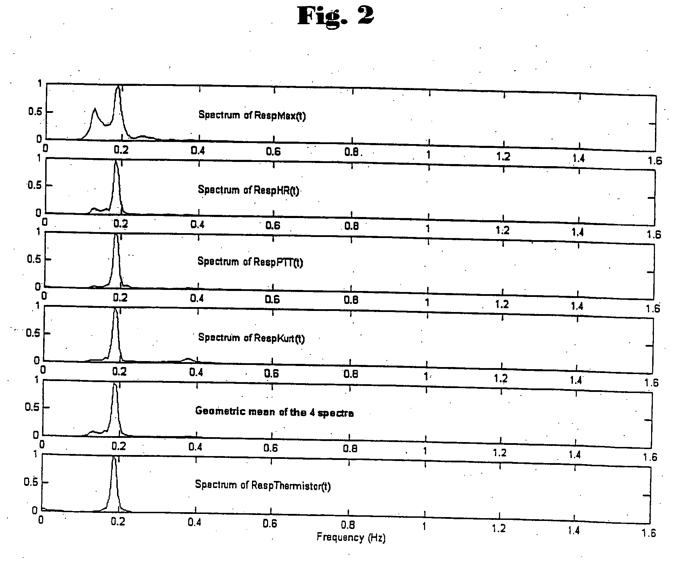

Aufgrund der Komplexität und Verschiedenheit der Übertragungswege können die Störrhythmen unterschiedlich in den extrahierten Atemsignalen $s_{hr}(t)$, $s_{max}(t)$, $s_{ptt}(t)$, $s_{kurt}(t)$ ausgeprägt seien. Fig. 1 und Fig. 2 zeigen vier solche Atemsignale im Zeit- bzw. im Frequenzbereich.

Die Signalauswertung zeigt weiterhin, dass die Ausprägungen der Störrhythmen in den vier Atemsignalen personenabhängig und zeitvariant sind. Aus diesem Grund ist es meistens schwierig, die Güte der extrahieren Atemsignale zu beurteilen. Beispielsweise kann man nicht einfach festlegen, dass $s_{hr}(t)$ definitiv besser oder schlechter als $s_{ptt}(t)$ ist.

Dem Grundgedanken der Methodenkombination im Zeit- oder Frequenzbereich liegt die oben genannte Beobachtung zu Grunde. Sie dient der Erhöhung der Zuverlässigkeit der aus dem EKG und PPG extrahierten Ateminformation.

Um z.B. vier unterschiedliche Methoden kombinieren zu können, müssen zuerst die 2 folgenden Schritte gemacht werden:

- Erfassen des EKG- und PPG-Signals für eine vorgegebene Zeitdauer T und Ermitteln der Herzfrequenz $hr(t)$, des PPG-Maximums max($t$), der Pulswellenlaufzeit $ptt(t)$ und des Kurtosiswerts $kurt(t)$ auf "beat-to-beat" Basis.

- Filtern der vier Signale mit Hilfe des Bandpasses 0,12 Hz ~ 0,42 Hz. Daraus ergeben sich die vier entsprechenden Atemsignale $s_{hr}(t)$, $s_{max}(t)$, $s_{ptt}(t)$ und $s_{kurt}(t)$.

4.1 Kombination im Zeitbereich

4.1.1 Ermittlung der momentanen Atemfrequenz

- lokale Maxima auffinden und ihren Zeitindex $t_{max}(n)$ in Sekunden speichern
- Berechnung der Atemfrequenz nach:

$$f(n) = \frac{60\,\text{sec}}{t_{max}(n) - t_{max}(n-1)} \text{ in Atemzüge/min}$$

- Ermittlung der momentanen Atemfrequenz aus den vier Atemsignalen

$f_{hr}(n)$ aus $s_{hr}(t)$
$f_{max}(n)$ aus $s_{max}(t)$
$f_{ptt}(n)$ aus $s_{ptt}(t)$
$f_{kurt}(n)$ aus $s_{kurt}(t)$

4.1.2 Kombination durch gewichtete Mittelung

Für eine gewichtete Mittelung werden zuerst die 4 gemessenen Atemfrequenzen mit einem Schätzwert der aktuellen Atemfrequenz verglichen und ihre Differenzen zum Schätzwert berechnet. Danach erfolgt die Berechnung der Gewichtfaktoren in Abhängigkeit von den Differenzen. Je größer die Differenz ist, desto kleiner ist der Gewichtsfaktor. Schließlich wird eine endgültige Atemfrequenz durch die gewichtete Mittelwertbildung festgelegt.

Im folgenden wird die gewichtete Mittelung näher beschrieben, wobei die letzte Atemfrequenz als der Schätzwert der aktuellen Atemfrequenz betrachtet wird.

1. Berechnung der Abweichung der momentanen Atemfrequenz von der letzten Atemfrequenz $f(n-1)$:

$$\sigma_{hr}^2 = \left[ f_{hr}(n) - f(n-1) \right]^2$$

$$\sigma_{max}^2 = \left[ f_{max}(n) - f(n-1) \right]^2$$

$$\sigma_{ptt}^2 = \left[ f_{ptt}(n) - f(n-1) \right]^2$$

$$\sigma_{kurt}^2 = \left[ f_{kurt}(n) - f(n-1) \right]^2$$

2. Berechnung des Gewichtfaktors:

$$k_{hr} = \frac{\sum - \sigma_{hr}^2}{3 \cdot \sum}$$

$$k_{max} = \frac{\sum - \sigma_{max}^2}{3 \cdot \sum}$$

$$k_{ptt} = \frac{\sum - \sigma_{ptt}^2}{3 \cdot \sum}$$

$$k_{kurt} = \frac{\sum - \sigma^2_{kurt}}{3 \cdot \sum}$$

wobei $\sum = \sigma^2_{hr} + \sigma^2_{max} + \sigma^2_{ptt} + \sigma^2_{kurt}$

3. Berechnung der aktuellen Atemfrequenz $f(n)$ durch gewichtete Mittelung nach:

$$f(n) = f_{hr}(n) \cdot k_{hr} + f_{max}(n) \cdot k_{max} + f_{ptt}(n) \cdot k_{ptt} + f_{kurt}(n) \cdot k_{kurt}$$

4. Initialisierung $f(0)$

- Initialisierung mit einem Festwert, z. B. 12 Atemzüge/min - normale Atemfrequenz für Erwachsene:

$$f(0) = 12 \text{ Atemzüge/min}$$

- Initialisierung mit dem arithmetischen Mittelwert der momentanen Atemfrequenzen:

$$f(0) = \frac{1}{4} \cdot \left[ f_{hr}(0) + f_{max}(0) + f_{ptt}(0) + f_{kurt}(0) \right]$$

- $f(0)$ ergibt sich aus einer mit Hilfe der Kombination im Frequenzbereich ermittelten Atemfrequenz.

5. Tabelle 2 zeigt einige Beispiele der gewichteten Mittelung

Tabelle 2: Beispiele für die gewichtete Mittelung

| letzter Wert $f(n-1)$ | $f_{hr}(n)$ | $f_{max}(n)$ | $f_{ptt}(n)$ | $f_{kurt}(n)$ | gewichteter Mittelwert $f(n)$ | arithmetischer Mittelwert |
|---|---|---|---|---|---|---|
| 12 | 15 | 15 | 15 | 15 | 15,0 | 15,0 |
| 12 | 12 | 13 | 11 | 8 | 11,9 | 11,0 |
| 12 | 13 | 11 | 6 | 8 | 10,6 | 9,5 |
| 12 | 11 | 8 | 7 | 6 | 8,4 | 8,0 |
| 12 | 9 | 8 | 7 | 4 | 7,5 | 7,0 |

4.1.3 Kombination durch Konsistenzprüfung - "Consensus Method"

Die vier Atemfrequenzen von $f_{hr}(n)$, $f_{max}(n)$, $f_{ptt}(n)$ und $f_{kurt}(n)$ werden untereinander auf Übereinstimmung mit Berücksichtigung einer vorgegebenen Toleranz geprüft. Danach errechnet sich in Abhängigkeit von der Anzahl der Übereinstimmungen eine endgültige Atemfrequenz über arithmetische oder gewichtete Mittelung aus den übereinstimmenden Atemfrequenzen. Je mehr Übereinstimmungen vorhanden sind, desto zuverlässiger ist die endgültige Atemfrequenz.

Die Konsistenzprüfung wird wie folgt näher beschrieben.

1. Man definiert eine Toleranz $\Delta$ als erlaubte Abweichung für die Prüfung der Übereinstimmung der Atemfrequenzen $f_{hr}(n)$, $f_{max}(n)$, $f_{ptt}(n)$ und $f_{kurt}(n)$, z.

$$B. \quad \Delta = 2 \text{ Atemzüge/min.}$$

Die Toleranz $\Delta$ kann von den vergangenen Messdaten abhängig sein. Beispielsweise kann sie von der letzten momentanen Atemfrequenz und/oder einer mittleren Atemfrequenz abhängig sein.

2. Berechnung der Abweichung zweier Atemfrequenzen nach

$$\Delta_{k-l} = \left| f_k(n) - f_l(n) \right|$$

Berechnen eines Konsistenzfaktors nach

konsistent: $\alpha_{k-l} = 1$, wenn $\Delta_{kl} \leq \Delta$
nicht konsistent: $\alpha_{k-l} = 0$, wenn $\Delta_{kl} > \Delta$

Damit ergeben sich insgesamt 6 Konsistenzfaktoren, die in Tabelle 3 zusammengefasst sind:

Tabelle 3: Konsistenzfaktoren

|  | $f_{hr}(n)$ | $f_{max}(n)$ | $f_{ptt}(n)$ | $f_{kurt}(n)$ |
|---|---|---|---|---|
| $f_{hr}(n)$ | 1 | $\alpha_{hr\text{-}max}$ | $\alpha_{hr\text{-}ptt}$ | $\alpha_{hr\text{-}kurt}$ |
| $f_{max}(n)$ |  | 1 | $\alpha_{ppg\text{-}ppt}$ | $\alpha_{ppg\text{-}kurt}$ |
| $f_{ptt}(n)$ |  |  | 1 | $\alpha_{ptt\text{-}kurt}$ |
| $f_{kurt}(n)$ |  |  |  | 1 |

3. Mindestens zwei von vier Atemfrequenzen müssen konsistent sein, um eine Atemfrequenz ermitteln zu können. Die Bestimmung der endgültigen Atemfrequenz erfolgt über eine gewichtete Mittelwertbildung.

4.2 Kombination im Frequenzbereich

Die Bildung eines geometrisch gemittelten Spektrums ist der Kernpunkt der Kombination im Frequenzbereich. Dadurch sollen die Störrhythmen in den Signalen ganz oder teilweise eliminiert werden. Diese Methode beruht auf der Beobachtung, dass einerseits die Störrhythmen sehr unterschiedlich und andererseits der Atemrhythmus relativ konsistent in den extrahierten Atemsignalen von $s_{hr}(t)$, $s_{max}(t)$, $s_{ptt}(t)$ und $s_{kurt}(t)$ ausgeprägt sind.

Die Methodenkombination im Frequenzbereich erfolgt über:

1. Die Signale von $s_{hr}(t)$, $s_{max}(t)$, $s_{ptt}(t)$ und $s_{kurt}(t)$ für ein gegebenes Zeitintervall werden durch z. B. FFT ("Fast Fourier Transformation") in den Frequenzraum transformiert und anschließend normiert. Daraus ergeben sich die entsprechen Spektren von $FT_{hr}(f)$, $FT_{max}(f)$, $FT_{ptt}(f)$ und $FT_{kurt}(f)$.

2. Der geometrische Mittelwert der Spektren wird berechnet durch:

$$FT_{mean}(f) = \left[ FT_{hr}(f) \cdot FT_{max}(f) \cdot FT_{ptt}(f) \cdot FT_{kurt}(f) \right]^{1/4}$$

3. Ermittlung einer mittleren Atemfrequenz aus $FT_{mean}(f)$ durch

a) z. B. Peak-Detektion oder

b) das gemittelte Spektrum $FT_{mean}(f)$ wird in den Zeitbereich rücktransformiert. Daraus ergibt sich ein zeitliches Atemsignal $s_{mean}(t)$, das teilweise oder ganz von Störrhythmen befreit ist. Aus $s_{mean}(t)$ kann die momentane Atemfrequenz nach der im Abschnitt 4.1.1 beschriebenen Methode ermittelt werden.

Im Vergleich zur Kombination im Zeitbereich weist die Kombination im Frequenzbereich den Nachteil auf, dass mehr Rechen- und Zeitaufwand in Anspruch genommen werden muss.

4.3. Ein konkretes Ausführungsbeispiel

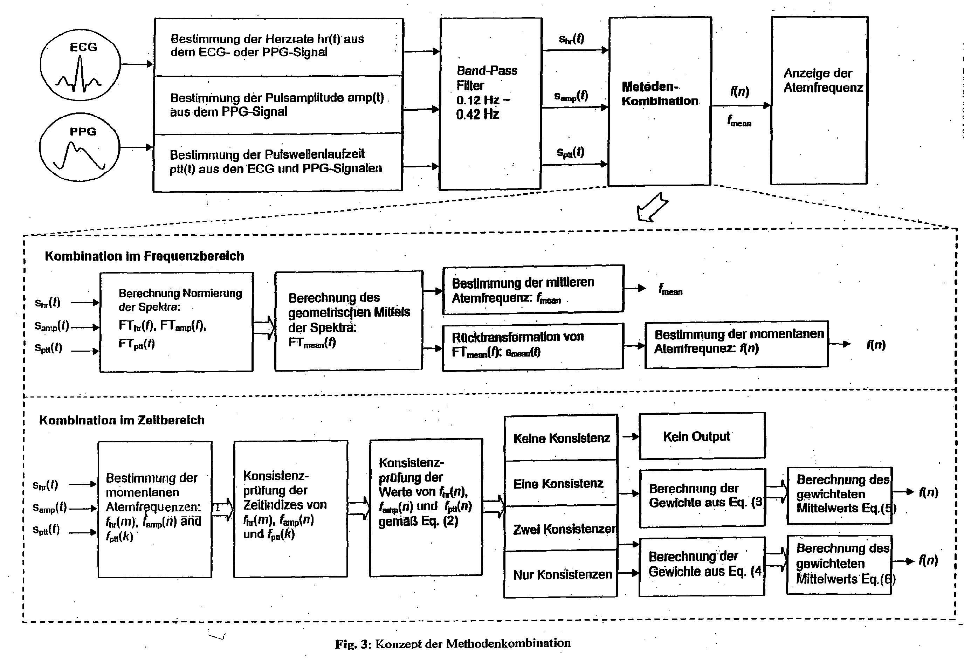

In dem konkreten Ausführungsbeispiel der erfindungsgemäßen Methodenkombination werden Signale aus drei unterschiedlichen Kanälen kombiniert, wobei alle drei oben beschriebenen Kombinationsmethoden, d.h. die Kombination durch gewichtete Mittelung, durch eine Konsistenzprüfung sowie durch eine Mittelung im Frequenzraum, zum Einsatz kommen. Ein Schema dieses Ausführungsbeispiels ist dabei in Fig. 3 zu sehen.

**Extrahierung der Ateminformation aus dem EKG und PPG**

1. Erfassen des EKG- und PPG-Signals für eine vorgegebene Zeitdauer T und Ermitteln der folgenden drei Atemsignale:

rr(t) or pp(t) - RR-Abstand aus dem EKG oder "Peak-to-Peak"-Abstand aus dem PPG
amp(t) - Pulsamplitude aus dem PPG Signal
ptt(t) - Pulswellenlaufzeit aus dem PPG Signal und dem EKG Signal

2. Filtern der drei Signale mit dem Bandpassfilter von 0,12 Hz ~ 0,42 Hz. Daraus ergeben sich

$s_{hr}(t)$ - Atemsignal aus der Variation der Herzrate rr(t) or pp(t)
$s_{amp}(t)$ - Atemsignal aus der Variation der Pulsamplitude amp(t)
$s_{ptt}(t)$ - Atemsignal aus der Variation der Pulswellenlaufzeit ptt(t)

**Kombination im Frequenzbereich**

Die Bildung des geometrisch gemittelten Spektrums ist der Kernpunkt in der Kombination im Frequenzbereich. Dadurch sollen die Störrhythmen, die innerhalb des Frequenzbereichs (0,12 Hz - 0,42Hz) des Bandpassfilters liegen und somit nicht durch den Filter eliminiert werden können, in den extrahierten Atemsignalen ganz oder teilweise eliminiert werden. Diese Methode beruht auf die Beobachtung, dass einerseits die Störrhythmen sehr unterschiedlich und andererseits der Atemrhythmus relativ konsistent in den extrahierten Atemsignalen von $s_{hr}(t)$, $s_{amp}(t)$ und $s_{ptt}(t)$ ausgeprägt sind.

Anhand der Abb. 3 wird die Methodenkombination im Frequenzbereich beispielsweise für $s_{hr}(t)$, $s_{amp}(t)$ und $s_{ptt}(t)$ erläutet. Sie erfolgt über:

1. Die Signale von $s_{hr}(t)$, $s_{amp}(t)$ und $s_{ptt}(t)$ für ein gegebenes Zeitintervall werden durch z.B. FFT ("Fast Fourier Transformation") in den Frequenzbereich transformiert und anschließend normiert. Daraus ergeben sich die entsprechenden Spektra von $FT_{hr}(f)$, $FT_{amp}(f)$ und $FT_{ptt}(f)$

2. Der geometrische Mittelwert der Spektra wird berechnet nach:

$$FT_{mean}(f) = \left[ FT_{hr}(f) \cdot FT_{amp}(f) \cdot FT_{ptt}(f) \right]^{1/3} \tag{1}$$

3. Ermittelung einer mittleren Atemfrequenz aus $FT_{mean}(f)$ durch z.B. Peak-Detektion oder

4. Das gemittelte Spektrum $FT_{mean}(f)$ wird in den Zeitbereich rücktransformiert. Daraus ergibt sich ein zeitliches Atemsignal $s_{mean}(t)$, das teilweise oder ganz von Störrhythmen befreit ist.

5. Aus $s_{mean}(t)$ kann die momentane Atemfrequenz nach der im Abschnitt 4.1.1 beschriebenen Methode ermittelt werden.

**Kombination im Zeitbereich**

1. Ermittlung der momentanen Atemfrequenz in Atemzüge/min:

$f_{hr}(m)$ aus $s_{hr}(t)$
$f_{amp}(n)$ aus $s_{amp}(t)$
$f_{ptt}(k)$ aus $s_{ptt}(t)$

2. Konsistenzprüfung für die Zeitindizes von m, n und k:

Sie müssen sich innerhalb eines vorgegebenen Zeitfensters befinden, sofern sie zu einer Atemaktivität bzw. einem Atemzug gehören. Für das Zeitfenster kann man beispielsweise 50% der aktuellen Atemperiode verwenden. Ist die Prüfung bestanden, werden die Atemfrequenzen erneut bezeichnet als $f_{hr}(n)$, $f_{amp}(n)$ und $f_{ptt}(n)$.

3. Konsistenzprüfung für die Werte der Atemfrequenzen von $f_{hr}(n)$, $f_{amp}(n)$ und $f_{ptt}(n)$ nach:

$$\left| f_A(m) - f_B(m) \right| \le th \tag{2}$$

wobei *A, B = hr, amp, ptt*
Beispielweise *th* = 2,5 Atemzüge/min oder *th* = 15% der letzten Atemfrequenz

Fortführung nach Prüfergebnis:

a) keine Konsistenz: CP = 0
b) eine Konsistenz: CP = 1, z.B. nur für $f_{amp}(n)$ und $f_{ptt}(n)$
c) zwei Konsistenzen: CP = 2, z.B. für sowohl $f_{amp}(n)$ und $f_{ptt}(n)$ als auch $f_{amp}(n)$ und $f_{ptt}(n)$

4. Berechnung der Gewichtsfaktoren, die auf der letzten Atemfrequenz aus der Kombination basieren.

a) Fall 1: CP = 0
Kein Gewichtsfaktor wird berechnet.
b) Fall 2: CP = 1

$$k_{amp} = \frac{\sum - e_{amp}^2}{\sum}$$

$$k_{ptt} = \frac{\sum - e_{ptt}^2}{\sum}$$

$$e_{amp} = f_{amp}(n) - f(n-1)$$

$$e_{ptt} = f_{ptt}(n) - f(n-1) \tag{3}$$

$$\sum = e_{amp}^2 + e_{ptt}^2$$

wobei *f(n-1)* - letzte gültige Atemfrequenz aus der Kombination
c) Fall 3: CP = 2

$$k_{hr} = \frac{\sum - e_{hr}^2}{2 \cdot \sum}$$

$$k_{amp} = \frac{\sum - e_{amp}^2}{2 \cdot \sum}$$

$$k_{ptt} = \frac{\sum - e_{ptt}^2}{2 \cdot \sum}$$

$$e_{hr} = f_{hr}(n) - f(n-1)$$

$$e_{amp} = f_{amp}(n) - f(n-1)$$

$$e_{ptt} = f_{ptt}(n) - f(n-1)$$

$$\sum = e_{ht}^2 + e_{amp}^2 + e_{ptt}^2$$

$$(4)$$

5. gewichtete Mittelung

    a) Fall 1: CP = 0
    keine Mittelung möglich => keine Ausgabe der Atemfrequenz

    b) Fall 2: CP = 1

$$f(n) = k_{amp} \cdot f_{amp}(n) + k_{ptt} \cdot f_{ptt}(n) \qquad (5)$$

    c) Fall 3: CP = 2

$$f(n) = k_{hr} \cdot f_{hr}(n) + k_{amp} \cdot f_{amp}(n) + k_{ptt} \cdot f_{ptt}(n) \qquad (6)$$

6. Initialisierung - Ermittelung des ersten Werts der Atemfrequenz $f(0)$

- Möglichkeit 1
  Bei einer bestandenen Konsistenzprüfung (CP $\geq$ 1) errechnet sich $f(0)$ als arithmetischer Mittelwert der konsistenten Atemfrequenzen

- Möglichkeit 2
  Man führt die Methodenkombination im Frequenzbereich durch und nimmt die daraus ermittelte mittlere Atemfrequenz als $f(0)$.

**Ergebnis**

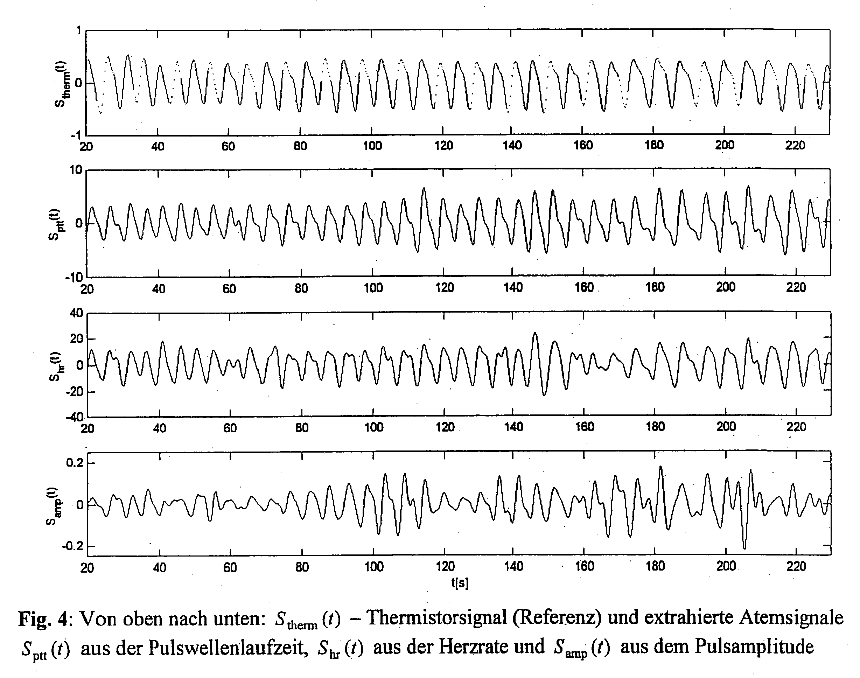

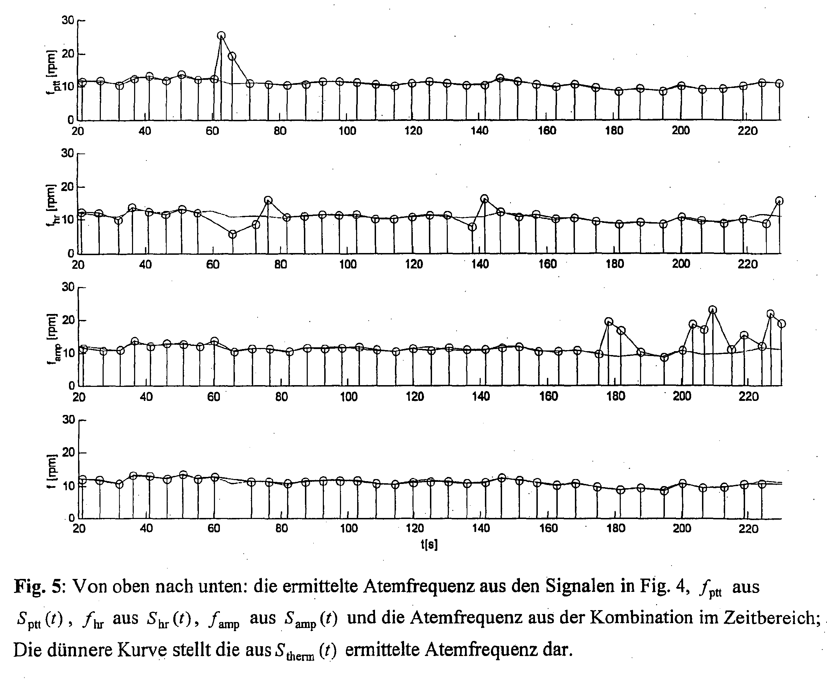

Abb. 4 stellt von oben nach unten das Thermistorsignal $s_{therm}(t)$ (Referenz), die extrahierten Atemsignale von $s_{ptt}(t)$ aus der Pulswellenlaufzeit, $s_{hr}(t)$ aus der Herzrate und $s_{amp}(t)$ aus der Pulsamplitude dar. Abb. 5 zeigt die aus den in Abb. 4 dargestellten Signalen ermittelten Atemfrequenzen und die Atemfrequenz aus der Kombination im Zeitbereich. Die dünnen Kurven in Abb. 5 zeigen die Atemfrequenz aus dem Thermistorsignal.

Aus Abb. 5 ist klar zu erkennen, dass die einzelnen Atemfrequenzen aus den jeweiligen extrahierten Atemsignalen an einigen Stellen von den Atemfrequenzen aus dem Thermistorsignal abweichen, z.B. $f_{ptt}$ zwischen 60 s und 70 s; $f_{hr}$ zwischen 60 s und 80 s, um 140 s, nach 220 s; $f_{amp}$ um 180 s, nach 200 s. Ganz im Gegenteil stimmt die Atemfrequenz aus der Kombination sehr gut mit der Atemfrequenz aus dem Thermistorsignal überein. Man erkennt auch, dass die Störungen der Atemfrequenz zwischen 60 s und 70 s durch die Kombination eliminiert werden. Die Ursache hierfür ist die Konsistenzprüfung, welche die gestörten Signale nicht bestanden haben.

4.4 Allgemeinheit der Methodenkombination

Die oben genannte Methodenkombination ist nicht auf Signale von $s_{hr}(t)$, $s_{max}(t)$, $s_{ptt}(t)$ und $s_{kurt}(t)$ beschränkt. Sie kann sowohl für aus dem EKG- und/oder PPG-Signal extrahierte Atemsignale als auch für Atemsignale, die mit anderen Sensoren/Methoden (z. B. Thermistor, Impedanzpneumographie, Induktionsplethysmographie) erfasst werden, verwendet werden.

Auch können die unterschiedlichen Alternativen der Methodenkombination wie z.B. die gewichtete Mittelwertbildung, die Konsistenzprüfung und die Kombination im Frequenzbereich wiederum miteinander kombiniert werden.

Die eingangs erwähnten unterschiedlichen Methoden zur Bestimmung der Atemfrequenz sind in folgenden Publikationen dargestellt, deren Inhalt in die vorliegende Anmeldung durch Bezugnahme aufgenommen wird:

[1] Association of the Advancement of Medical Instrumentation (AAMI): Apnea Monitoring by Means of Thoracic Impedance Pneumography, AAMI, Arlington, VA, 1989,

[2] Hirsch JA, Bishop B.: Respiratory Sinus Arrhytmia in Humans: How breathing pattern modulates Heart rate, Am J Physiol. 1981 Oct; 241 (4): H620-9

[3] Anders Johansson, Per Ake Öberg und Gunnar Sedin: Monitoring of Heart and Respiratory Rates in Newborn Infants using a new Photoplethymographic Technique, Journal of Clinical Monitoring and Computing, 15: 461-467,199

[4] Shuxue Ding, Xin Zhu, Wenxi Chen und Daming Wei: Derivation of Respiratory Signal from Single-Channel ECG Based on Source Statistics, International Journal of Bioelectromagnetism, Vol.6, No. 1, 2004

[5] Wei Zhang, DE 10014077A1 "Verfahren und Vorrichtung zur Bestimmung der Atemaktivität eines Lebewesens, Anmeldetag 22.3.2000

Abkürzungen:

EKG:    Elektrokardiogramm - Aufzeichnung der Herzaktivität durch die Erfassung der herzerregungsabhängigen Potentialdifferenzen an der Körperoberfläche.

PPG:    Photoplethysmogramm - Aufzeichnung des Blutvolumens mit Hilfe eines optoelektrischen Messverfahrens

PTT:    Pulswellenlaufzeit - die Zeit, welche eine Pulswelle braucht, um sich entlang einer Arterie von einer (herznahen) Position A bis zu einer (peripheren) Position B zu bewegen

RSA:    Respiratorische Sinusarrhythmie - atembedingte Änderung in der Herzrate

Resp:   Respiration

sBP:    systolischer Blutdruck

HR:     Herzrate

FFT:    "Fast Fourier Transformation"

**Patentansprüche**

1.  Verfahren zur Bestimmung der Atemfrequenz eines Patienten, mit den Schritten:

    - Bestimmen von mindestens zwei zeitabhängigen Atemsignalen $s_i(t)$ ($i$ = 1, 2, ...) durch mindestens zwei unterschiedliche Messmethoden;
    - Bestimmen der sich aus den mindestens zwei zeitabhängigen Atemsignalen $s_i(t)$ ($i$ = 1, 2,...) ergebenden jeweiligen momentanen Atemfrequenzen $f_i(n)$ ($i$ = 1, 2, ...)

- Bestimmen einer mittleren Atemfrequenz $f(n)$ durch eine gewichtete Mittelung der Atemfrequenzen $f_i(n)$ ($i$ = 1, 2,...),

**dadurch gekennzeichnet,**

**dass** die Gewichte $k_i(n)$ ($i$ = 1, 2, ...) der einzelnen Atemfrequenzen $f_i(n)$ ($i$ = 1, 2, ...) von einer Differenz zwischen den jeweiligen Atemfrequenzen $f_i(n)$ ($i$ = 1, 2, ...) und einem Schätzwert $f_s(n)$ abhängen, der auf Grundlage der mindestens zwei zeitabhängigen Atemsignale $s_i(t)$ ($i$ = 1, 2, ...) bestimmt wird.

2. Verfahren gemäß Anspruch 1, wobei der Schätzwert $f_s(n)$ auf Grundlage einer vorangegangenen bereits bestimmten mittleren Atemfrequenz $f(n$-1) bestimmt wird.

3. Verfahren gemäß Anspruch 1, wobei der Schätzwert $f_s(n)$ insbesondere zur Initialisierung durch eine Kombination von Frequenzinformationen aus mindestens zwei zeitabhängigen Atemsignalen $s_i(t)$ ($i$ = 1, 2,...) oder durch Bilden eines Mittelwertes der aktuellen Atemfrequenzen $f_i(n)$ ($i$ = 1, 2,...) bestimmt wird.

4. Verfahren gemäß Anspruch 1, wobei die mindestens zwei zeitabhängigen Atemsignale $s_i(t)$ ($i$ = 1, 2,...) aus gemessenen physiologischen Signalen ermittelt werden.

5. Verfahren gemäß Anspruch 4, wobei die gemessenen physiologischen Signale eine Auswahl aus folgenden Signalen bilden:

   - Bioimpedanzsignal,
   - Herzratenvariabilitätssignal,
   - photoplethysmographisches Signal (PPG-Signal),
   - Quellenstatistiksignal des EKGs,
   - Pulswellenlaufzeitsignal (PTT-Signal).

6. Verfahren gemäß Anspruch 4, wobei die Ermittlung der mindestens zwei zeitabhängigen Atemsignale $s_i(t)$ ($i$ = 1, 2,...) aus den gemessenen physiologischen Signalen durch einen Bandpassfilter geschieht.

7. Verfahren gemäß Anspruch 6, wobei der Bandpassfilter Frequenzen in einen Bereich von circa 0,12 Hz bis 0,42 Hz passieren lässt.

8. Verfahren gemäß Anspruch 1, wobei die Bestimmung der jeweiligen momentanen Atemfrequenzen $f_i(k_i)$, insbesondere von $f_{hr}(m)$, $f_{amp}(n)$ und $f_{ptt}(k)$, aus den zeitabhängigen Atemsignalen $s_i(t)$ ($i$ = 1, 2,...) durch Bestimmung der Zeitindizes $t_{max}(k_i)$, insbesondere $t_{max}(m)$, $t_{max}(n)$ und $t_{max}(k)$, der Maxima der zeitabhängigen Atemsignale $s_i(t)$ ($i$ = 1, 2,...) geschieht.

9. Verfahren gemäß Anspruch 8, wobei eine Konsistenzprüfung für die Zeitindizes $k_i$ ($i$ = 1, 2,...), insbesondere m, n und k, der momentanen Atemfrequenzen erfolgt.

10. Verfahren gemäß Anspruch 1, wobei eine Konsistenzprüfung der Atemfrequenzen $f_i(n)$ ($i$ = 1, 2,...) durchgeführt wird.

11. Verfahren gemäß Anspruch 10, wobei die Konsistenzprüfung durch einen Vergleich der der Atemfrequenzen $f_i(n)$ ($i$ = 1, 2,...) untereinander erfolgt.

12. Verfahren gemäß Anspruch 11, wobei die Differenzen zwischen den jeweiligen Atemfrequenzen $f_i(n)$ ($i$ = 1, 2, ...) bestimmt und mit einer erlaubten Toleranz $\Delta$ verglichen werden.

13. Verfahren gemäß Anspruch 10, wobei zu der gewichteten Mittelung der Atemfrequenzen $f_i(n)$ ($i$ = 1, 2,...) nur solche Atemfrequenzen herangezogen werden, welche die Konsistenzprüfung bestehen.

14. Verfahren gemäß einem der vorangegangenen Ansprüche, wobei die Signalgüte insbesondere über eine Konsistenzprüfung bestimmt und gegebenenfalls angezeigt wird.

15. Verfahren gemäß Anspruch 1, welches umfasst

   - Erzeugung von mindestens zwei Frequenzsignalen
   $FT_i(f)$ ($i$ = 1, 2, ...) durch Transformation von mindestens zwei zeitabhängigen Atemsignalen $s_i(t)$ ($i$ = 1, 2,...) in den Frequenzraum

- Bestimmen eines Frequenzsignals *FT(f)* durch eine Kombination der Frequenzsignale *FT_i(f)* ($i$ = 1, 2,...), wobei eine Atemfrequenz *f* auf Grundlage des Frequenzsignals *FT(f)* bestimmt wird.

**16.** Verfahren gemäß Anspruch 15, wobei das Frequenzsignals *FT(f)* durch eine Mittelung der Frequenzsignale *FT_i(f)* ($i$ = 1, 2,...) bestimmt wird.

**17.** Verfahren gemäß Anspruch 15, wobei die Atemfrequenz *f* durch Peak-Detektion des Frequenzsignals *FT(f)* bestimmt wird.

**18.** Verfahren gemäß Anspruch 15, wobei die Atemfrequenz *f* durch Rücktransformation des Frequenzsignals *FT(f)* und eine Auswertung des sich ergebenden Signals *s(t)* bestimmt wird.

**19.** Verfahren gemäß Anspruch 1 und 15, wobei die Atemfrequenz *f* zur Initialisierung der gewichteten Mittelung verwendet wird.

**20.** Verfahren gemäß einem der vorangegangenen Ansprüche, wobei die mindestens zwei zeitabhängigen Atemsignale $s_i(t)$ ($i$ = 1, 2,...) aus einem PPG-Signal und einem EKG-Signal gewonnenen werden.

**21.** Verfahren gemäß einem der vorangegangenen Ansprüche, wobei die mindestens zwei zeitabhängigen Atemsignale $s_i(t)$ ($i$ = 1, 2,...) eine Auswahl aus folgenden Signalen bilden:

- ein aus der Herzfrequenz bestimmtes Atemsignal $s_{HR}(t)$,
- ein aus dem PPG-Signal bestimmtes Atemsignal $s_{PPG}(t)$,
- ein aus dem PTT-Signal bestimmtes Atemsignal $s_{PTT}(t)$,
- ein aus der Kurtosis des EKG-Signals bestimmtes Atemsignal $s_{kurt}(t)$.

**22.** Verfahren gemäß Anspruch 21, wobei mindestens drei Atemsignale verwendet werden.

**23.** Vorrichtung zur Bestimmung der Atemfrequenz eines Patienten mittels eines Verfahrens nach einem der vorangegangenen Ansprüche, mit einer separaten Sensoreinheit zur Messung der physiologischen Signale, aus welchen die mindestens zwei zeitabhängigen Atemsignale bestimmt werden können, und einer Recheneinheit zur Auswertung der von der Sensoreinheit übertragenen Daten.

**24.** Vorrichtung nach Anspruch 23, wobei die von der Sensoreinheit erzeugten Daten drahtlos an die Recheneinheit übertragen werden.

**25.** Vorrichtung nach Anspruch 23 oder 24, wobei die Sensoreinheit am Handgelenk des Patienten befestigt wird.

**26.** Vorrichtung nach einem der Ansprüche 23-25, wobei die mindestens zwei zeitabhängigen Atemsignale in der Sensoreinheit aus den physiologischen Signalen bestimmt werden und daraufhin an die Recheneinheit übermittelt werden.

**27.** Vorrichtung nach einem der Ansprüche 23-26, mit Sensoren zur Messung des EKG-Signals und des PPG-Signals.

**28.** Vorrichtung nach Anspruch 27, wobei aus dem EKG-Signal und dem PPG-Signal die Herzrate, die Pulsamplitude und die Pulswellenlaufzeit bestimmt werden.

**29.** Vorrichtung nach einem der Ansprüche 23-28, wobei die Recheneinheit Teil eines medizinischen Geräts, insbesondere eines medizinischen Gerätes zur extrakorporalen Blutbehandlung ist.

**30.** Vorrichtung nach einem der Ansprüche 23-28, wobei die Recheneinheit Teil eines Rechnernetzwerks ist.

**Claims**

**1.** A method of determining the respiratory rate of a patient comprising the steps:

- determining at least two time dependent respiratory signals $s_i(t)$ ($i$ = 1, 2,...) by at least two different measuring methods;
- determining the resulting respective instantaneous respiratory rates $f_i(n)$ ($i$ = 1, 2,...) from the at least two time dependent respiratory signals $s_i(t)$ ($i$ = 1, 2, ...)
- determining an average respiratory rate $f(n)$ by a weighted averaging of the respiratory rates $f_i(n)$ ($i$ = 1, 2, ...), **characterized in that**
the weightings $k_i(n)$ ($i$ = 1, 2,...) of the individual respiratory rates $f_i(n)$ ($i$ = 1, 2, ...) depend on a difference between the respective respiratory rates $f_i(n)$ ($i$ = 1, 2, ...) and an estimate $f_s(n)$ determined on the basis of the at least two time-dependent respiratory signals $s_i(t)$ ($i$ = 1, 2,...).

2. A method in accordance with claim 1, wherein the estimate $f_s(n)$ is determined on the basis of a preceding, already determined average respiratory rate $f(n-1)$.

3. A method in accordance with claim 1, wherein the estimate $f_s(n)$ is determined, in particular for initialization by a combination of rate information from at least two time dependent respiratory signals $s_i(t)$ ($i$ = 1, 2,...) or by forming an average of the current respiratory rates $f_i(n)$ ($i$ = 1, 2, ...).

4. A method in accordance with claim 1, wherein the at least two time dependent respiratory signals $s_i(t)$ ($i$ = 1, 2,...) are determined from measured physiological signals.

5. A method in accordance with claim 4, wherein the measured physiological signals form a selection from the following signals:

   - bioimpedance signal;
   - heart rate variability signal;
   - photoplethysmographic signal (PPG signal);
   - statistical source signal of the ECG;
   - pulse wave transit time signal (PTT signal).

6. A method in accordance with claim 4, wherein the determination of the at least two time dependent respiratory signals $s_i(t)$ ($i$ = 1, 2,...) takes place from the measured physiological signals by a band pass filter.

7. A method in accordance with claim 6, wherein the band pass filter allows frequencies in a range from approx. 0.12 Hz to 0.42 Hz to pass.

8. A method in accordance with claim 1, wherein the determination of the respective instantaneous respiratory rates $f_i(k_i)$, in particular of $f_{hr}(m)$, $f_{amp}(n)$ and $f_{ptt}(k)$, from the time dependent respiratory signals $s_i(t)$ ($i$ = 1, 2,...) takes place by determining the time indices $t_{max}(k_i)$, in particular $t_{max}(m)$, $t_{max}(n)$ and $t_{max}(k)$, of the maxima of the time dependent respiratory signals $s_i(t)$ ($i$ = 1, 2, ...).

9. A method in accordance with claim 8, wherein a consistency check takes place for the time indices $k_i$ ($i$ = 1, 2,...), in particular m, n and k, of the instantaneous respiratory rates.

10. A method in accordance with claim 1, wherein a consistency check of the respiratory rates $f_i(n)$ ($i$ = 1, 2, ...) is carried out.

11. A method in accordance with claim 10, wherein the consistency check takes place by a comparison of the respiratory rates $f_i(n)$ ($i$ = 1, 2,...) among one another.

12. A method in accordance with claim 11, wherein the differences between the respective respiratory rates $f_i(n)$ ($i$ = 1, 2, ...) are determined and are compared with a permitted tolerance $\Delta$.

13. A method in accordance with claim 10, wherein only those respiratory rates are used for the weighted averaging of the respiratory rates $f_i(n)$ ($i$ = 1, 2,...) which pass the consistency check.

14. A method in accordance with one of the preceding claims, wherein the signal quality is in particular determined via a consistency check and is optionally displayed.

**15.** A method, in particular in accordance with claim 1, comprising

- the generation of at least two frequency signals $FT_i(f)$ ($i$ = 1, 2,...) by transformation of at least two time dependent respiratory signals $s_i(t)$ ($i$ = 1, 2,...) into the frequency domain
- determination of a frequency signal $FT(f)$ by a combination of the frequency signals $FT_i(f)$ ($i$ = 1, 2,...), wherein a respiratory rate $f$ is determined on the basis of the frequency signal $FT(f)$.

**16.** A method in accordance with claim 15, wherein the frequency signal $FT(f)$ is determined by an averaging of the frequency signals $FT_i(f)$ ($i$ = 1, 2, ...).

**17.** A method in accordance with claim 15, wherein the respiratory rate $f$ is determined by peak detection of the frequency signal $FT(f)$.

**18.** A method in accordance with claim 15, wherein the respiratory rate $f$ is determined by back transformation of the frequency signal $FT(f)$ and an evaluation of the resulting signal $s(t)$.

**19.** A method in accordance with claims 1 and 15, wherein the respiratory rate $f$ is used for the initialization of the weighted averaging.

**20.** A method in accordance with one of the preceding claims, wherein the at least two time dependent respiratory signals $s_i(t)$ ($i$ = 1, 2,...) are acquired from a PPG signal and an ECG signal.

**21.** A method in accordance with one of the preceding claims, wherein the at least two time dependent respiratory signals $s_i(t)$ ($i$ = 1, 2,...) form a selection from the following signals:

- a respiratory signal determined from the heart rate $s_{HR}(t)$,
- a respiratory signal determined from the PPG signal $s_{PPG}(t)$,
- a respiratory signal determined from the PTT signal $s_{PTT}(t)$,
- a respiratory signal determined from the kurtosis of the ECG signal $s_{kurt}(t)$.

**22.** A method in accordance with claim 21, wherein at least three respiratory signals are used.

**23.** An apparatus for determining the respiratory rate of a patient by means of a method in accordance with one of the preceding claims, comprising a separate sensor unit for measuring the physiological signals from which the at least two time dependent respiratory signals can be determined and a processing unit for the evaluation of the data transmitted by the sensor unit.

**24.** An apparatus in accordance with claim 23, wherein the data generated by the sensor unit are transmitted to the processing unit in a wireless manner.

**25.** An apparatus in accordance with either of claims 23 or 24, wherein the sensor unit is fastened to the patient's wrist.

**26.** An apparatus in accordance with one of the claims 23 to 25, wherein the at least two time dependent respiratory signals are determined from the physiological signals in the sensor unit and are thereupon transmitted to the processing unit.

**27.** An apparatus in accordance with one of the claims 23 to 26 comprising sensors for the measurement of the ECG signal and the PPG signal.

**28.** An apparatus in accordance with claim 27, wherein the heart rate, the pulse amplitude and the pulse wave transit time are determined from the ECG signal and the PPG signal.

**29.** An apparatus in accordance with one of the claims 23 to 28, wherein the processing unit is part of a medical device, in particular of a medical device for extracorporeal blood treatment.

**30.** An apparatus in accordance with one of the claims 23 to 28, wherein the processing unit is part of a computer network.

**Revendications**

1. Procédé de détermination de la fréquence respiratoire d'un patient, comprenant les étapes consistant à :

   - déterminer au moins deux signaux de respiration dépendants du temps $s_i(t)$ ($i$ = 1, 2, ...) par au moins deux méthodes de mesure différentes ;
   - déterminer les fréquences respiratoires $f_i(n)$ ($i$ = 1, 2, ...) momentanées respectives résultant des au moins deux signaux de respiration dépendants du temps $s_i(t)$ ($i$ = 1, 2, ...)
   - déterminer une fréquence respiratoire moyenne $f(n)$ par le calcul pondéré d'une moyenne des fréquences respiratoires $f_i(n)$ ($i$ = 1, 2, ...),
   **caractérisé en ce que**
   les poids $k_i(n)$ ($i$ = 1, 2, ...) des différentes fréquences respiratoires $f_i(n)$ ($i$ = 1, 2, ...) dépendent d'une différence entre les fréquences respiratoires $f_i(n)$ ($i$ = 1, 2, ...) respectives et une valeur estimée $f_s(n)$, qui est déterminée sur la base des au moins deux signaux de respiration dépendants du temps $s_i(t)$ ($i$ = 1, 2, ...).

2. Procédé selon la revendication 1, dans lequel la valeur estimée $f_s(n)$ est déterminée sur la base d'une fréquence respiratoire moyenne précédente $f(n$ -1) déjà déterminée.

3. Procédé selon la revendication 1, dans lequel la valeur estimée $f_s(n)$ est déterminée, en particulier pour l'initialisation, par une combinaison d'informations de fréquence d'au moins deux signaux de respiration dépendants du temps $s_i(t)$ ($i$ = 1, 2, ...) ou par la formation d'une valeur moyenne des fréquences respiratoires $f_i(n)$ ($i$ = 1, 2, ...) actuelles.

4. Procédé selon la revendication 1, dans lequel les au moins deux signaux de respiration dépendants du temps $s_i(t)$ ($i$ = 1, 2, ...) sont déterminés à partir de signaux physiologiques mesurés.

5. Procédé selon la revendication 4, dans lequel les signaux physiologiques mesurés forment une sélection des signaux suivants :

   - signal de bio-impédance,
   - signal de variabilité de fréquence cardiaque,
   - signal photopléthysmographique (signal PPG),
   - signal de statistique de source de l'ECG,
   - signal du temps de propagation des ondes pulsées (signal PTT).

6. Procédé selon la revendication 4, dans lequel la détermination des au moins deux signaux de respiration dépendants du temps $s_i(t)$ ($i$ = 1, 2, ...) est effectuée par un filtre passe-bande à partir des signaux physiologiques mesurés.

7. Procédé selon la revendication 6, dans lequel le filtre passe-bande laisse passer des fréquences dans une plage allant d'environ 0,12 Hz à 0,42 Hz.

8. Procédé selon la revendication 1, dans lequel la détermination des fréquences respiratoires momentanées respectives $f_i(k_i)$, en particulier de $f_{hr}(m)$, $f_{amp}(n)$ et $f_{ptt}(k)$, est effectuée à partir des signaux de respiration dépendants du temps $s_i(t)$ ($i$ = 1, 2, ...) par la détermination des indices de temps $t_{max}$ $(k_i)$, en particulier $t_{max}$ ($m$), $t_{max}$ ($n$) et $t_{max}$ ($k$), des maxima des signaux de respiration dépendants du temps $s_i(t)$ ($i$ = 1, 2, ...).

9. Procédé selon la revendication 8, dans lequel un contrôle de cohérence pour les indices de temps $k_i$($i$ = 1, 2, ...), en particulier m, n et k, des fréquences respiratoires momentanées est effectué.

10. Procédé selon la revendication 1, dans lequel un contrôle de cohérence des fréquences respiratoires $f_i(n)$ ($i$ = 1, 2, ...) est exécuté.

11. Procédé selon la revendication 10, dans lequel le contrôle de cohérence est effectué par une comparaison des fréquences respiratoires $f_i(n)$ ($i$ = 1, 2, ...) entre elles.

12. Procédé selon la revendication 11, dans lequel les différences entre les fréquences respiratoires $f_i(n)$ ($i$ = 1, 2, ...) respectives sont déterminées et comparées à une tolérance $\Delta$ autorisée.

13. Procédé selon la revendication 10, dans lequel, pour le calcul pondéré de la moyenne des fréquences respiratoires

$f_i(n)$ (i = 1, 2,...), seules les fréquences respiratoires qui passent avec succès le contrôle de cohérence sont prises en compte.

14. Procédé selon l'une des revendications précédentes, dans lequel la qualité du signal est déterminée en particulier par un contrôle de cohérence et elle est indiquée le cas échéant.

15. Procédé selon la revendication 1, qui comprend

    - la génération d'au moins deux signaux de fréquence $FT_i(f)$ (i = 1, 2,...) par la transformation d'au moins deux signaux de respiration dépendants du temps $s_i(t)$ (i = 1, 2, ...) dans l'espace de fréquence
    - la détermination d'un signal de fréquence $FT(f)$ par une combinaison des signaux de fréquence $FT_i(f)$ (i = 1, 2, ...), dans lequel une fréquence respiratoire f est déterminée sur la base du signal de fréquence $FT(f)$.

16. Procédé selon la revendication 15, dans lequel le signal de fréquence $FT(f)$ est déterminé par un calcul de la moyenne des signaux de fréquence $FT_i(f)$ (i = 1, 2, ...).

17. Procédé selon la revendication 15, dans lequel la fréquence respiratoire f est déterminée par détection de crête du signal de fréquence $FT(f)$.

18. Procédé selon la revendication 15, dans lequel la fréquence respiratoire f est déterminée par transformation inverse du signal de fréquence $FT(f)$ et une évaluation du signal $s(t)$ obtenu.

19. Procédé selon la revendication 1 et 15, dans lequel la fréquence respiratoire f est utilisée pour l'initialisation du calcul pondéré de la moyenne.

20. Procédé selon l'une des revendications précédentes, dans lequel les au moins deux signaux de respiration dépendants du temps $s_i(t)$ (i = 1, 2, ...) sont obtenus à partir d'un signal PPG et d'un signal ECG.

21. Procédé selon l'une des revendications précédentes, dans lequel les au moins deux signaux de respiration dépendants du temps $s_i(t)$ (i = 1, 2, ...) forment une sélection des signaux suivants :

    - un signal de respiration déterminé à partir de la fréquence cardiaque $s_{HR}(t)$,
    - un signal de respiration déterminé à partir du signal PPG $s_{PPG}(t)$,
    - un signal de respiration déterminé à partir du signal PTT $s_{PTT}(t)$,
    - un signal de respiration déterminé à partir du kurtosis du signal ECG $s_{kurt}(t)$.

22. Procédé selon la revendication 21, dans lequel au moins trois signaux de respiration sont utilisés.

23. Dispositif de détermination de la fréquence respiratoire d'un patient au moyen d'un procédé selon l'une des revendications précédentes, comprenant une unité de détection séparée destinée à mesurer les signaux physiologiques, à partir desquels les au moins deux signaux de respiration dépendants du temps peuvent être déterminés, et une unité de calcul destinée à évaluer les données transmises par l'unité de détection.

24. Dispositif selon la revendication 23, dans lequel les données générées par l'unité de détection sont transmises sans fil à l'unité de calcul.

25. Dispositif selon la revendication 23 ou 24, dans lequel l'unité de détection est fixée au poignet du patient.

26. Dispositif selon l'une des revendications 23 à 25, dans lequel les au moins deux signaux de respiration dépendants du temps sont déterminés dans l'unité de détection à partir des signaux physiologiques et sont ensuite transmis à l'unité de calcul.

27. Dispositif selon l'une des revendications 23 à 26, comprenant des capteurs destinés à mesurer le signal ECG et le signal PPG.

28. Dispositif selon la revendication 27, dans lequel la fréquence cardiaque, la pression pulsée et le temps de propagation d'onde pulsée sont déterminés à partir du signal ECG et du signal PPG.

**29.** Dispositif selon l'une des revendications 23 à 28, dans lequel l'unité de calcul fait partie d'un appareil médical, en particulier d'un appareil médical pour le traitement extracorporel du sang.

**30.** Dispositif selon l'une des revendications 23 à 28, dans lequel l'unité de calcul fait partie d'un réseau informatique.

# Fig. 1

# Fig. 2

**Fig. 3:** Konzept der Methodenkombination

**Fig. 4:** Von oben nach unten: $S_{therm}(t)$ – Thermistorsignal (Referenz) und extrahierte Atemsignale $S_{ptt}(t)$ aus der Pulswellenlaufzeit, $S_{hr}(t)$ aus der Herzrate und $S_{amp}(t)$ aus dem Pulsamplitude

**Fig. 5:** Von oben nach unten: die ermittelte Atemfrequenz aus den Signalen in Fig. 4, $f_{ptt}$ aus $S_{ptt}(t)$, $f_{hr}$ aus $S_{hr}(t)$, $f_{amp}$ aus $S_{amp}(t)$ und die Atemfrequenz aus der Kombination im Zeitbereich; Die dünnere Kurve stellt die aus $S_{therm}(t)$ ermittelte Atemfrequenz dar.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20050027205 A

- DE 10014077 A1, Wei Zhang

**In der Beschreibung aufgeführte Nicht-Patentliteratur**

- Association of the Advancement of Medical Instrumentation (AAMI): Apnea Monitoring by Means of Thoracic Impedance Pneumography. AAMI, 1989
- **HIRSCH JA ; BISHOP B.** Respiratory Sinus Arrhytmia in Humans: How breathing pattern modulates Heart rate. *Am J Physiol.,* Oktober 1981, vol. 241 (4), H620-9

- **ANDERS JOHANSSON ; PER AKE ÖBERG ; GUNNAR SEDIN.** Monitoring of Heart and Respiratory Rates in Newborn Infants using a new Photoplethymographic Technique. *Journal of Clinical Monitoring and Computing,* vol. 15, 461-467
- **SHUXUE DING ; XIN ZHU ; WENXI CHEN ; DAMING WEI.** Derivation of Respiratory Signal from Single-Channel ECG Based on Source Statistics. *International Journal of Bioelectromagnetism,* 2004, vol. 6 (1